# EUROPÄISCHE PATENTSCHRIFT

(11) **EP 2 033 591 B1**
(45) Veröffentlichungstag und Bekanntmachung des Hinweises auf die Patenterteilung: **14.08.2013**
(21) Anmeldenummer: 08075750.3
(22) Anmeldetag: 04.09.2008
(51) Int. Cl.: A61B 19/00, A61C 1/00, A61B 17/00

(54) **Verfahren zum Anmelden von kabellosen elektrischen Stelleinrichtungen an einem medizinischen Gerät**
Method for pairing a wireless remote control with a medical device
Procédé de reconnaissance mutuelle d'une télécommande sans fil et d'un appareil médical

(30) Priorität: 04.09.2007 DE 102007042389
(43) Veröffentlichungstag der Anmeldung: 11.03.2009
(73) Patentinhaber: Carl Zeiss Meditec AG, 07745 Jena (DE)
(72) Erfinder: Göttlich, Joachim, 73447 Oberkochen (DE)
(74) Vertreter: Theobald, Andreas

(56) Entgegenhaltungen:
- WO-A-96/06499
- WO-A-98/02860
- WO-A-2005/001790
- WO-A-2007/047128
- US-A1- 2006 116 667

## Beschreibung

Während einer Operation (OP) kann eine Vielzahl medizinischer Geräte zum Einsatz kommen. Diese Geräte können unmittelbar zum Operieren dienen, wie etwa Laser, oder den behandelnden Arzt während der OP unterstützen, wie etwa Operationsmikroskop. Das Bedienen derartiger medizinischer Geräte durch einen operierenden Arzt oder einen Assistenten erfolgt häufig mit dem Fuß. Dies hat einerseits seinen Grund darin, dass der operierende Arzt und der Assistent möglichst die Hände zum Operieren frei haben und nicht auch zum Einstellen des medizinischen Geräts benutzen müssen. Zum anderen sind Bedienelemente medizinischer Geräte oft nur schwierig zu sterilisieren, so dass ein Berühren mit der Hand das Risiko einer Kontamination in sich birgt.

Wenn in einem Operationssaal ein Fußschaltpult (FSP) oder eine andere elektrische Stelleinrichtung zur Steuerung eines medizinischen Gerätes, bspw. zur Steuerung des Stativs eines Operationsmikroskops, eingesetzt werden, soll das entsprechende Gerät nur die Funktion ausführen, die genau von diesem Fußschaltpult bzw. genau von dieser Stelleinrichtung initiiert wird. Dies kann durch eine kabelgebundene Verbindung zwischen der Stelleinrichtung (im Folgenden soll unter Stelleinrichtung jede Art eines Bedienelementes für das medizinische Gerät verstanden werden, also auch bspw. ein Fußschaltpult) erreicht werden. Eine Kabelverbindung hat jedoch den Nachteil, dass sie einerseits eine Stolpergefahr mit sich bringen kann und andererseits sich fahrbare medizinische Geräte in Kabeln verfangen können. Das Risiko nimmt umso mehr zu, je mehr medizinische Geräte und zugeordnete Stelleinrichtungen in einem Operationssaal zur Anwendung kommen.

Man ist daher bestrebt, kabelgebundene Stelleinrichtungen durch kabellose Stelleinrichtungen zu ersetzen, in denen die Kommunikation zwischen der Stelleinrichtung und dem medizinischen Gerät kabellos erfolgt, bspw. über Funksignale, Infrarotsignale, Ultraschallsignale, etc. Wenn derartige Kabellose Stelleinrichtungen zur Steuerung eines medizinischen Geräts eingesetzt werden, soll das entsprechende Stativ nur die Funktionen ausführen, die von einer Stelleinrichtung initiiert werden, die dem medizinischen Gerät zuvor durch Ausführen eine speziellen Anmeldeprozedur, auch "Pairing" genannt, zugeordnet wurde. Würden Funktionen von einer anderen in der Nähe befindlichen und zu einem anderen Gerät gehörenden Stelleinrichtung ausgelöst werden, könnte dies zu Komplikationen führen.

Ein kabelloses Fußschaltpult für ein medizinisches Behandlungsgerät ist in WO 2007/084668 A2 beschrieben. Zum Anmelden des Fußschaltpultes wird eine auf dem Fußschaltpult angeordnete Synchronisationstaste betätigt, die eine Anmeldeprozedur am medizinischen Gerät auslöst. Während der Anmeldeprozedur werden ldentifikationsadressen der Stelleinrichtung und des medizinischen Gerätes ausgetauscht.

Eine kabellose Stelleinrichtung für ein medizinisches Gerät ist ebenfalls in WO 2004/019751 A2 beschrieben. Auch bei dieser Stelleinrichtung wird die Anmeldeprozedur von der Stelleinrichtung ausgelöst. Im Unterschied zum Fußschaltpult in WO 2007/084668 A2 erfolgt die Anmeldeprozedur nicht über den eigentlichen kabellosen Signalübertragungspfad sondern über einen zweiten, speziell für die Anmeldeprozedur vorgesehenen Signalübertragungspfad mit geringer Reichweite. Zum Durchführen der Anmeldeprozedur muss daher das Fußschaltpult an das medizinische Gerät angenähert werden, da sonst auf Grund der geringen Reichweite des speziellen Signalübertragungspfades ein Durchführen der Anmeldeprozedur nicht möglich wäre. Dadurch soll die Gefahr einer versehentlichen Anmeldung an einem falschen, weiter entfernten medizinischen Gerät verringert werden.

Die US 2006/0116667 A1 beschreibt ein medizinisches System mit einer zentralen Steuereinheit, die drahtlos beispielsweise mit einem Fußschaltpult kommuniziert. Zur Kommunikation weisen beide jeweils einen Funkempfänger und einen entsprechenden Sender auf. Weiterhin weist das Fußschaltpult einen RFID-Chip und die zentrale Steuereinheit ein RFID-Leser auf. Zur Synchronisation des Fußschaltpultes mit der zentralen Steuereinheit wird das Fußschaltpult in die Nähe desjenigen Bereichs der zentralen Steuereinheit gebracht, in dem sich der RFID-Leser befindet. Dadurch wird der Identifikationscode des Fußschaltpultes an die zentrale Steuereinheit übertragen. Alternative Einrichtungen zum Durchführen des Anmeldevorgangs sind ebenfalls beschrieben, nämlich Strichcodeleser, Infrarotsender und -empfänger und akustische Systeme.

Die WO 98/02860 A1 beschreibt das Übersenden eines ID-Codes von einer Mastereinheit zu einer Slaveeinheit, wobei der Vorgang von der Mastereinheit mit reduzierter Sendeleistung durchgeführt wird. Auch die Anwendung im Rahmen einer Fernbedienung ist erwähnt.

Die WO 2007/047128 A1 beschreibt ein Verfahren zum Ermöglichen einer zuverlässigen Kommunikation zwischen drahtlos miteinander verbundenen medizinischen Vorrichtungen. Eine zuverlässige Kommunikation wird dabei durch die parallele Verwendung mehrerer Datenkanäle erreicht. In dem Dokument ist außerdem das Durchführen eines Anmeldevorgangs eines Steuergeräts an einer Vorrichtung erwähnt, ohne jedoch im Detail beschrieben zu sein.

Die WO 96/06499 A2 beschreibt ein Verfahren zum Übertragen von geschützten Daten von einer Fernbedienung an ein Gerät. Ein in der Fernbedienung vorhandener Sender kann dabei mit sehr geringer Leistung arbeiten, um beispielsweise im Rahmen eines Authorisierungsprozesses die geschützten Daten nur an das in der Nähe befindlichen Gerät zu übertragen. Gegenüber diesem Stand der Technik ist es Aufgabe der vorliegenden Erfindung, ein vorteilhaftes Anmeldeverfahren zum Anmelden von kabellosen elektrischen Stelleinrichtungen an einem medizinischen Gerät über einen kabellosen Kommunikationskanal zur Verfügung zu stellen.

Diese Aufgabe wird durch das Verfahren zum Anmelden von kabellosen elektrischen Stelleinrichtungen an einem medizinischen Gerät über einen kabellosen Kommunikationskanal nach Anspruch 1 gelöst. Die abhängigen Ansprüche enthalten vorteilhafte Ausgestaltungen des Verfahrens.

Das erfindungsgemäße Verfahren zum Anmelden von kabellosen elektrischen Stelleinrichtungen an einem medizinischen Gerät über einen kabellosen Kommunikationskanal umfasst die Schritte: Initiieren des Anmeldevorgangs für eine kabellose elektrische Stelleinrichtung an einem medizinischen Gerät und Durchführen einer Anmeldeprozedur, in der die Stelleinrichtung dem medizinischen Gerät zugeordnet wird. Die Initiierung des Anmeldevorgangs erfolgt im erfindungsgemäßen Verfahren vom medizinischen Gerät aus. Die Sendeleistung des medizinischen Gerätes und/oder der Stelleinrichtung ist während des Anmeldevorgangs verringert. Das Initiieren des Anmeldevorgangs löst ein Absenken der Sendeleistung des Senders des medizinischen Gerätes und ein anschließendes Aussenden eines Aufrufs zum Durchführen der Anmeldeprozedur mit abgesenkter Sendeleistung aus. Nach dem Empfang eines Aufrufs zur Durchführen der Anmeldeprozedur durch eine Stelleinrichtung wird die Sendeleistung des Senders der entsprechenden Stelleinrichtung verringert, bevor die Stelleinrichtung eine Antwort auf den Aufruf sendet und die Anmeldeprozedur durchgeführt wird.

Dadurch, dass der Anmeldevorgang vom medizinischen Gerät initiiert wird und nicht wie in Stand der Technik üblich von der Stelleinrichtung, liegt die Initiative für den Anmeldevorgang beim medizinischen Gerät. Dadurch kann die Stelleinrichtung nur immer genau an dem medizinischen Gerät angemeldet werden, welches einen Anmeldevorgang initiiert hat. Andere medizinische Geräte, die keinen Anmeldevorgang initiiert haben, stehen für den Anmeldevorgang nicht zur Verfügung, so dass Fehlanmeldungen vermieden werden können.

Wenn zwei oder mehr medizinische Geräte derart nahe beieinander verwendet werden, dass ihre Funkreichweite im regulären Betrieb überlappt, und von zwei medizinischen Geräten mit überlappender Reichweite des kabellosen Kommunikationskanals gleichzeitig ein Anmeldevorgang initiiert wird kann es dennoch vorkommen, dass eine Stelleinrichtung am falschen medizinischen Gerät anmeldet. An dieser Stelle sei erwähnt, dass der Anmeldevorgang in der Regel durch betätigen einer Taste des Nutzers vorgenommen wird und es der Nutzer daher in der Hand hat, die Anmeldevorgänge an den medizinischen Geräten nacheinander durchzuführen, so dass eine Fehlanmeldung nicht erfolgen kann. Falls aber dennoch ein gleichzeitiges Initiieren eines Anmeldevorgangs erfolgt, können Fehlanmeldungen dadurch vermieden werden, dass die Sendeleistung des medizinischen Gerätes und/oder der Stelleinrichtung während des Anmeldevorgangs verringert ist. Durch das Verringern der Sendeleistung kann erreicht werden, dass die bei regulärer Sendeleistung des kabellosen Übertragungskanals überlappenden Sendebereiche nach der Reduzierung der Sendeleistung nicht mehr überlappen. Insbesondere kann die Sendeleistung soweit reduziert werden, dass eine Kommunikation zwischen dem medizinischen Gerät und der Stelleinrichtung nur im unmittelbaren Nahbereich möglich ist.

Das Initiieren des Anmeldevorgangs löst ein Absenken der Sendeleistung des Senders des medizinischen Gerätes und ein anschließendes Aussenden eines Aufrufs zum Durchführen der Anmeldeprozedur mit abgesenkter Sendeleistung aus. Es können dann nur solche Stelleinrichtungen den Aufruf zum Durchführen der Anmeldeprozedur empfangen, die sich im Nahbereich zum medizinischen Gerät befinden. Es ist dann insbesondere auch möglich, als Aufruf zum Durchführen der Anmeldeprozedur einen Sammelaufruf zu senden (broadcast).

Nach dem Empfang eines Aufrufs zur Durchführen der Anmeldeprozedur durch eine Stelleinrichtung die Sendeleistung des Senders der entsprechenden Stelleinrichtung verringert, bevor die Stelleinrichtung eine Antwort auf den Aufruf sendet und die Anmeldeprozedur durchgeführt wird. Durch das Absenken der Sendeleistung des Senders der Stelleinrichtung lässt sich eine Fehlanmeldung auch dann zuverlässig vermeiden, wenn ein zweites medizinisches Gerät vorhanden ist, das ebenfalls einen Anmeldevorgang initiiert hat und sich in Senderreichweite der unverringerten Sendeleistung, nicht aber in Senderreichweite der verringerten Sendeleistung befindet. Durch geeignete Abstände zwischen einer Stelleinrichtung und den medizinischen Geräten und/oder durch einen geeigneten Grad an Absenkung der Sendeleistung lässt sich erreichen, dass die Stelleinrichtung nur an dem nächstliegenden medizinischen Gerät angemeldet wird.

Wenn das Verringern der Sendeleistung und insbesondere das Antworten auf den Aufruf nur dann erfolgt, wenn eine Anmeldefreigabe durch betätigen einer Freigabetaste der Stelleinrichtung erfolgt, kann vermieden werden, dass Stelleinrichtungen, die sich innerhalb der reduzierten Senderreichweite um das medizinische Gerät befinden, aber nicht an diesem angemeldet werden sollen, fälschlicherweise am medizinischen Gerät angemeldet werden. Zusätzlich oder alternativ ist es möglich, dass eine bereits bestehende Zuordnung der Stelleinrichtung aufgehoben wird, wenn diese auf den Aufruf antwortet. Diese Maßnahme verhindert, dass eine Stelleinrichtung gleichzeitig bei zwei medizinischen Geräten angemeldet werden kann. Es liegt dadurch immer eine eindeutige Zuordnung der Stelleinrichtung zu einem bestimmten medizinischen Gerät vor.

Es ist außerdem vorteilhaft, wenn der Anmeldevorgang vom medizinischen Gerät beendet wird, wenn innerhalb einer vorgegebenen Zeitspanne keine Stelleinrichtung auf den Aufruf des medizinischen Gerätes antwortet. Da das medizinische Gerät in dieser Ausgestaltung des Verfahrens nicht dauerhaft im Anmeldemodus verbleibt, ist eine Anmeldung nach Ablauf der vorgegebenen Zeitspanne nicht mehr möglich. Bei mehreren medizinischen Geräten können daher Anmeldevorgänge so durchgeführt werden, dass ein Anmeldevorgang an einem weiteren medizinischen Gerät nicht initiiert wird, bevor die vorgegebene Zeitspanne abgelaufen ist. Auf diese Weise steht immer nur ein medizinisches Gerät für den Anmeldevorgang zur Verfügung, was Fehlanmeldungen zuverlässig vermeiden lässt.

Während der Anmeldeprozedur, also nach dem Senden des Aufrufs durch das medizinische Gerät und der Beantwortung des Aufrufs durch die Stelleinrichtung, werden Identifikationscodes, bspw. die Seriennummern der an der Anmeldeprozedur beteiligten Geräte, ausgetauscht. Die Identifikationscodes können im medizinischen Gerät und/oder in der Stelleinrichtung in einem nicht flüchtigen Speicher gespeichert werden. Die Zuordnung bleibt dann in dem entsprechenden medizinischen Gerät bzw. der entsprechenden Stelleinrichtung auch dann erhalten, wenn das Gerät bzw. die Stelleinrichtung ausgeschaltet wird oder die Kommunikationsverbindung unterbrochen wird, bspw. weil sie soweit voneinander entfernt werden, dass sie sich nicht mehr im wechselseitigen Sendebereich befinden.

Um das Zuordnen mehrerer Stelleinrichtungen an ein medizinisches Gerät zu ermöglichen, kann die Zuordnung bereits am medizinischen Gerät angemeldeter Stelleinrichtungen im medizinischen Gerät erhalten bleiben, wenn eine weitere Stelleinrichtung am medizinischen Gerät angemeldet wird. In diesem Fall ist es vorteilhaft, wenn die Zuordnung aller am medizinischen Gerät angemeldeten Stelleinrichtungen auf einen Aufhebebefehl hin aufgehoben werden kann. In diesem Fall werden die Identifikationscodes aus dem Speicher des medizinischen Geräts gelöscht. Nach dem Aufheben aller Zuordnungen besteht dann die Möglichkeit, Zuordnungen komplett neu zu erstellen. Das Aufheben der Zuordnung kann aber auch dann erfolgen, wenn die erste im Rahmen des Anmeldevorgangs anzumeldende Stelleinrichtung angemeldet wird. Die Zuordnung der Stelleinrichtungen bleibt dann bis zur Durchführung eines neuen Anmeldeverfahrens erhalten.

Nach Abschluss der Anmeldeprozedur wird die Sendeleistung des medizinischen Geräts und/oder der Stelleinrichtung vorteilhafterweise wieder auf ihren Standardwert erhöht, so dass die angemeldete Stelleinrichtung auch außerhalb des Nahbereichs des medizinischen Geräts Verwendung finden kann.

Das erfindungsgemäße Verfahren eignet sich insbesondere zum Anmelden von kabellosen elektrischen Stelleinrichtungen an medizinischen Stativen, wie bspw. Stativen für Operationsmikroskope. Die Stelleinrichtungen können hierbei insbesondere als Fußschaltpulte ausgebildet sein.

Weitere Merkmale, Eigenschaften und Vorteile der vorliegenden Erfindung ergeben sich aus der nachfolgenden Beschreibung eines Ausführungsbeispieles unter Bezugnahme auf die beiliegenden Figuren.
Figur 1 zeigt ein Stativ mit einer Mikroskophalterung und einem daran angeordneten Operationsmikroskop als Beispiel für ein medizinisches Gerät.
Figur 2 zeigt die Freiheitsgrade, die das Stativ und die Halterung aus Figur 1 zur Verfügung stellen.
Figur 3 zeigt ein Flussdiagram, welches das Verfahren zum Anmelden der kabellosen Stelleinrichtung am medizinischen Gerät auf Seiten des medizinischen Geräts darstellt.
Figur 4 zeigt ein Flussdiagram, das das Verfahren zum Anmelden der Stelleinrichtung am medizinischen Gerät auf Seiten der Stelleinrichtung darstellt.

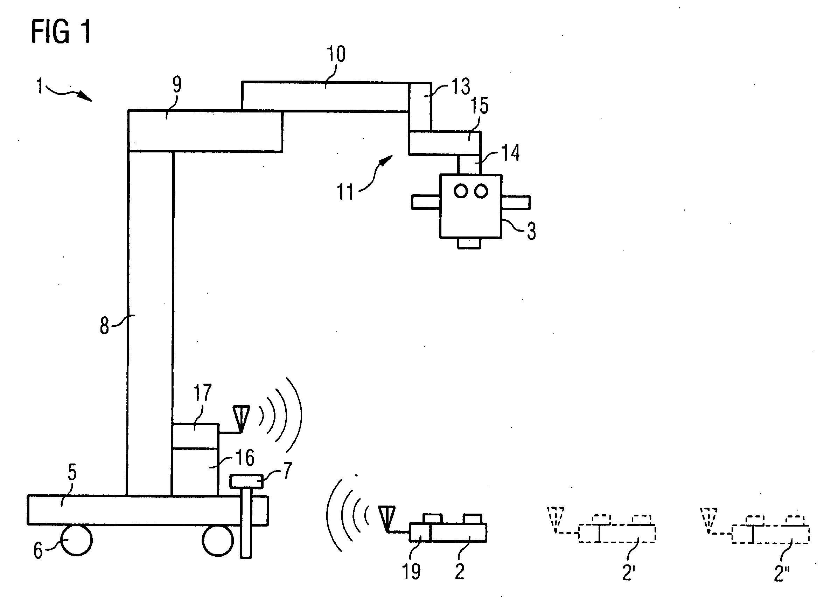

Ein Stativ 1 mit einem daran befestigten Mikroskop 3, das im hier ein Operationsmikroskop ist, ist in Fig.1 als ein Beispiel für ein medizinisches Gerät, das mit wenigstens einem kabellosen, bspw. auf Bluetooth basierenden Fußschaltpult 2 als Stelleinrichtung ausgestattet ist, dargestellt. Das Stativ 1 ruht auf einem Stativfuß 5, an dessen Unterseite Rollen 6 vorhanden sind, die ein Verfahren des Stativs 1 ermöglichen. Um ein ungewolltes Verfahren des Stativs 1 zu verhindern, besitzt der Stativfuß 5 außerdem eine Fußbremse 7.

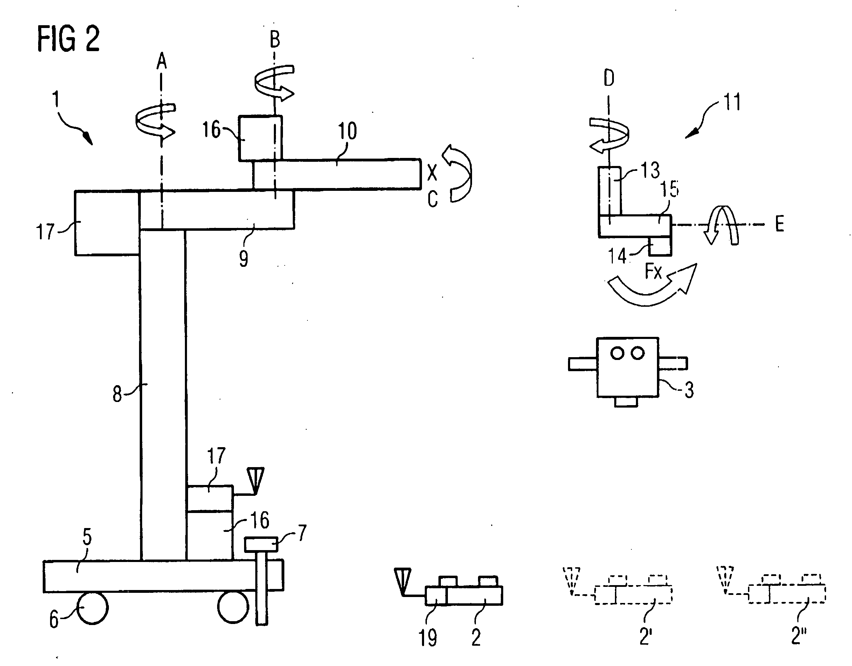

Das eigentliche Stativ 1 umfasst als Stativglieder eine höhenverstellbare Stativsäule 8, einen Tragarm 9, einen Federarm 10, und eine Mikroskopaufhängung 11, welche ihrerseits ein Verbindungselement 13, einen Schwenkarm 15 und einen Haltearm 14 umfasst. Die Freiheitsgrade, welche die Stativglieder zum Positionieren des Operationsmikroskops 3 zur Verfügung stellen und von denen wenigstens ein Teil durch das Fußschaltpult 2 gesteuert werden kann, sind in Fig. 2 gezeigt. Der Tragarm 9 ist an seinem einen Ende um eine Achse A drehbar mit der Stativsäule 8 verbunden. Am anderen Ende des Tragarms 9 ist ein Ende des Federarms 10 um eine zur Achse A parallele Achse B drehbar befestigt, so das der Tragarm 9 und der Federarm 10 einen Gelenkarm bilden. Das andere Ende des Federarms 10 ist von einem Kippmechanismus gebildet (nicht dargestellt), an dem die Mikroskopaufhängung 11 befestigt ist und der ein Verkippen der Mikroskopaufhängung 11 um die Achse C ermöglicht.

Die Mikroskopaufhängung 11 weist eine Drehachse D, eine Schwenkachse E sowie eine Kippachse F auf, um die sich das Mikroskop drehen, schwenken bzw. verkippen lässt. Mit einem Verbindungselement 13 ist die Mikroskopaufhängung 11 am äußeren Ende des Federarms 10 um die Drehachse D drehbar befestigt. Die Drehachse D erstreckt sich entlang des Verbindungselementes 13. An das Verbindungselement 13 schließt sich ein Schwenkarm 15 an, mit dessen Hilfe sich das Mikroskop 3, genauer gesagt ein am Schwenkarm 15 angebrachter Haltearm 14, an dem das Mikroskop 3 mittels einer Mikroskophalterung (nicht dargestellt) befestigt ist, um die Schwenkachse E schwenken lässt. Die Schwenkachse E erstreckt sich durch den Schwenkarm 15. Der Winkel zwischen Schwenkarm 15 und Verbindungselement 13, d.h. der Winkel zwischen der Schwenkachse E und der Drehachse D, kann mittels einem zwischen dem Verbindungsteil 13 und dem Schwenkarm 15 angeordneten Verstellmechanismus variiert werden.

Durch den Haltearm 14 verläuft senkrecht zur Darstellungsebene die Kippachse F, die ein Verkippen des Operationsmikroskops 3 ermöglicht. Das Operationsmikroskop 3 ist mittels einer nicht dargestellten Mikroskophalterung am Haltearm 14 befestigt.

Die Freiheitsgrade der Mikroskopaufhängung 11 sowie die Einstellmöglichkeiten des Operationsmikroskops 3, bspw. Fokussierung, Schärfe, Vergrößerungsfaktor, etc, können ebenfalls über ein kabelloses Stellglied 2', 2" einstellbar sein. Dabei ist es möglich, eine Stelleinrichtung für das Gesamtsystem aus Stativ, Halterung und Mikroskop vorzusehen oder für einzelne Geräte des Gesamtsystems individuelle Stelleinrichtungen vorzusehen, bspw. getrennte Fußschaltpulte für Stativ 1, Halterung 11 und Mikroskop 3.

Am Stativ 1 ist außerdem eine Steuereinheit 16 zum Steuern des Stativs 1 und/oder der Halterung 11 und/oder des Operationsmikroskops 3 vorhanden. Die Steuereinheit 16 basiert auf derselben kabellosen Übertragungstechnik wie das Fußschaltpult 2 und weist einen Sender/Empfänger 17 auf, der im vorliegenden Beispiel bluetoothfähig ist. Auch das Fußschaltpult 2 weist entsprechend einen bluetoothfähigen Sender/Empfänger 19 auf.

Um ein ungewolltes Verstellen des Mikroskops 3 aus einer gewählten Position zu verhindern, sind die Stativglieder bzw. die Gelenke zwischen den Stativgliedern mit Bremsen (nicht dargestellt) versehen, welche nach dem Positionieren des Mikroskops 3 fixiert werden. Als Bremsen kommen sowohl manuell als auch elektrisch zu betätigende Bremsen in Frage.

Um sicher zu stellen, dass das Stativsystem mit dem Operationsmikroskop 3 nur von dem richtigen Fußschaltpult 2 Steuerbefehle entgegennimmt, muss das entsprechende Fußschaltpult 2 am Stativsystem angemeldet werden. Falls das Stativ 1, die Halterung 11 oder das Mikroskop 3 ein eigenes Fußschaltpult aufweisen, sind für alle drei Geräte getrennte Anmeldungsvorgänge durchzuführen.

Die Anmeldung und Zuordnung bspw. eines Fußschaltpultes 2 oder einer anderen Stelleinrichtung beim Stativ 1 erfolgt für den Anwender in einfacher Weise und ist in sicherer Weise durchführbar. Ein genau definierter Ablauf, bei dem die Sendeleistung der beteiligten Funkmodule auf ein Minimum herabgesetzt wird, um die Zuordnung nur im Nahbereich zu ermöglichen, sorgt dafür, dass nur eine beabsichtigte Zuordnung (Pairing) möglich ist. Diese Zuordnung bleibt dann bis zum nächsten Pairingvorgang beibehalten. Ein Stativ führt nur Befehle aus, die von einem zugeordneten Fußschaltpult bzw. einer anderen zugeordneten Stelleinrichtung erhält. Einander zugeordnete Einheiten können zudem durch zusätzlich angebrachte farbliche Kennungen auch visuell voneinander unterscheidbar gemacht werden.

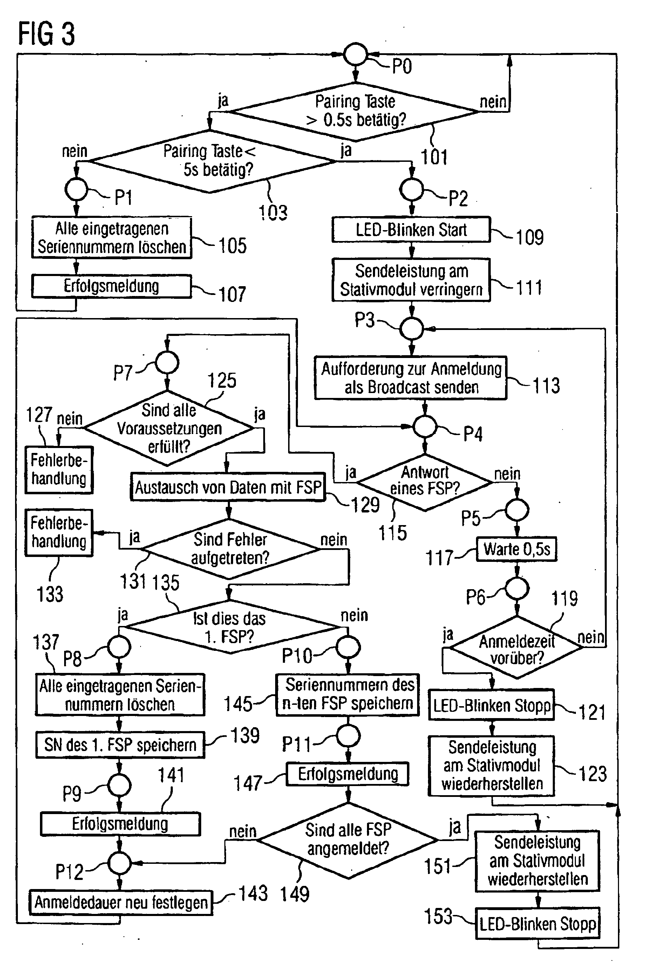

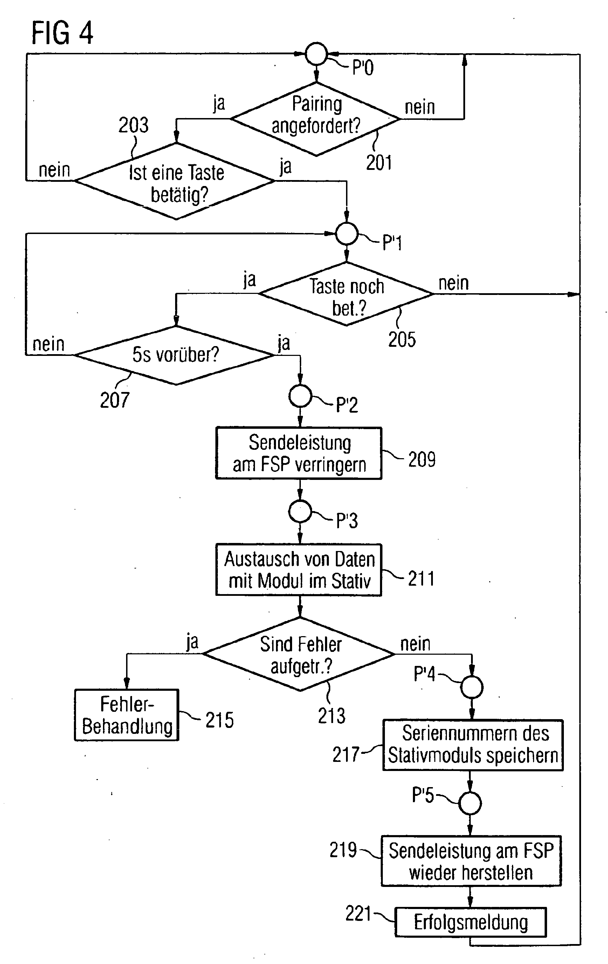

Der Ablauf des Anmeldeverfahrens wird nachfolgend mit Bezug auf die Figuren 3 und 4 am Beispiel des Anmeldens eines Fußschaltpultes 2 am Stativ 1 beschrieben. Figur 3 zeigt hierbei den in der Steuerung 16 des Stativs ablaufenden Teil des Anmeldevorgangs, während Figur 4 den im Fußschaltpult 2 ablaufenden Teil des Anmeldevorgangs darstellt.

Zu Beginn des Anmeldeverfahrens befindet sich die Steuereinheit 16 des Stativs 1 im Prozess P0, der einen Leerlaufprozess darstellt. Wenn in diesem Zustand eine so genannte Pairingtaste betätigt wird, wird das Anmeldeverfahren zum Anmelden des Fußschaltpultes 2 am Stativ 1, das so genannte Pairing, eingeleitet. In einem ersten Schritt 101 erfolgt dann eine Abfrage, ob die Pairingtaste länger als eine halbe Sekunde betätigt worden ist. Wenn die Antwort auf diese Anfrage nein lautet, so verbleibt das System im Leerlaufprozess. Falls die Abfrage mit ja beantwortet wird, schreitet das Verfahren zu Schritt 103 voran, in dem eine Abfrage dahingehend erfolgt, ob die Pairingtaste weniger als fünf Sekunden lang betätigt worden ist. Falls diese Abfrage verneint wird, geht das Verfahren in den Aufhebungsprozess P1 über, in dem in Schritt 105 alle bestehenden Zuordnungen von Stelleinrichtungen 2 zum Stativ 1 aufgehoben werden, indem alle beim Stativ eingetragenen Seriennummern von Stelleinrichtungen gelöscht werden. Nach der Ausgabe einer Erfolgsmeldung in Schritt 107 kehrt der Steuereinheit dann in den Leerlaufprozess P0 zurück.

Falls in Schritt 103 die Frage, ob die Pairingtaste weniger als fünf Sekundenlang betätigt worden ist, mit ja beantwortet wird, so schreitet das Verfahren zum Prozess P2 fort, in dem eine LED Anzeige am Stativ 1 in Schritt 109 zum Blinken veranlasst wird und die Sendeleistung des Senders 17 am Stativ in Schritt 111 verringert wird. Danach schreitet das Verfahren zum Prozess P3 voran, in dem in Schritt 113 ein Aufruf zur Anmeldung am Stativ 1 gesendet wird. Die Prozesse P2 du P3 dienen zum initiieren des Anmeldevorgangs und können insgesamt als Initiierungsprozess für die Anmeldeprozedur angesehen werden.

Nach der Initiierung des Anmeldevorgangs schreitet das Verfahren zum Prozess P4 fort, der einen Warteprozess darstellt, in dem auf eine Antwort einer Stelleinrichtung, also bspw. des Fußschaltpultes 2, gewartet wird. Wenn in Schritt 115 des Warteprozesses P4 festgestellt wird, dass keine Antwort einer Stelleinrichtung eingegangen ist, schreitet das Verfahren zum Prozess P5 fort, in dem in Schritt 117 eine Wartezeit von einer halben Sekunde verstrichen lassen wird, bevor das Verfahren zum Prozess P6 voranschreitet. Im Prozess P6 erfolgt dann eine Abfrage dahingehend, ob eine vorgegebene Zeitspanne verstrichen ist (Schritt 119), d.h. eine vorgegebene Anmeldezeitdauer verstrichen ist. Wird dies verneint, so kehrt das Verfahren zu Prozess P3 zurück, in dem dann die Aufforderung zur Anmeldung erneut als Sammelaufruf versendet wird. Wird in Schritt 119 hingegen festgestellt, dass die vorgegebene Zeitspanne verstrichen ist, so schreitet das Verfahren zu Schritt 121 fort, in dem das Blinken der LED am Stativ 1 beendet wird, und dann zu Schritt 123, in dem die Sendeleistung des Senders 17 am Stativ 1 wieder auf seinen Ausgangswert angehoben wird. Danach kehrt das Verfahren in den Leerlaufprozess P0 zurück.

Falls hingegen in Schritt 115 festgestellt wird, dass das Fußschaltpult 2, auf die Aufforderung zur Anmeldung hin geantwortet hat, schreitet das Verfahren zu Prozess P7 fort. Im Prozess P7 erfolgt zuerst in Schritt 125 eine Abfrage dahingehend, ob alle Voraussetzungen für das Durchführen der Anmeldeprozedur erfüllt sind. Wenn dies verneint wird, schreitet das Verfahren zu Schritt 127 fort, in dem eine Fehlerbehandlung durchgeführt werden kann. Sind dagegen alle Voraussetzungen für das Durchführen der Anmeldeprozedur erfüllt, so schreitet der Prozess P7 zu Schritt 129 voran, in dem die Steuereinheit 16 des Stativs 1 Daten mit dem Fußschaltpult 2 austauscht. Im Rahmen dieses Datenaustausches werden auch die Seriennummern zwischen dem Fußschaltpult 2 und der Steuereinheit 16 des Stativs 1 ausgetauscht. Die Zuordnung des Fußschaltpultes 2 zum Stativ 1 erfolgt dann anhand der ausgetauschten Seriennummern. In Schritt 131 wird dann überprüft, ob bei dem Datenaustausch Fehler aufgetreten sind. Falls dies der Fall ist, schreitet der Prozess P7 zu Schritt 133 fort, in dem eine Fehlerbehandlung durchgeführt werden kann. Sind keine Fehler aufgetreten, so erfolgt in Schritt 135 eine Abfrage, ob das Fußschaltpult 2 die erste Stelleinrichtung ist, für die die Anmeldeprozedur durchgeführt wird. Ist dies der Fall, so schreitet das Verfahren zu Prozess P8 voran.

Im Prozess P8 werden in einem ersten Schritt (Schritt 137) alle zuvor eingetragenen Seriennummern gelöscht und dann die Seriennummer des Fußschaltpultes 2 in einem nichtpflichtigen Speicher gespeichert (Schritt 139). Durch das Speichern in einem nichtpflichtigen Speicher bleibt die Seriennummer auch dann gespeichert, wenn die Bluetoothverbindung zwischen dem Fußschaltpult 2 und der Steuereinheit 16 oder die Stromversorgung eines der beiden Geräte unterbrochen wird. Dadurch bleibt die Zuordnung auch während eines Verlustes der Bluetoothverbindung bzw. der Stromversorgung erhalten. Wenn die Bluetoothverbindung bzw. die Stromversorgung wiederhergestellt ist, ist daher im Unterschied zum Stand der Technik keine erneute Anmeldung notwendig.

Nach der Speicherung der Seriennummer schreitet das Verfahren zu Prozess P9 voran, in dem in Schritt 141 eine Erfolgsmeldung ausgegeben wird. Danach wird in einem Prozess P12 die vorgegebene Zeitspanne für das Antworten auf den Sammelaufruf wieder hergestellt, und das Verfahren kehrt zu Prozess P4 zurück, in dem es auf eine Antwort auf den Sammelaufruf wartet.

Falls in Schritt 135 festgestellt wird, dass im Rahmen des Anmeldeverfahrens bereits eine Stelleinrichtung am Stativ 1 angemeldet worden ist, schreitet das Verfahren statt zu Prozess P8 zu Prozess P10 voran, in dem die Seriennummer des aktuell angemeldeten Fußschaltpultes als N-te Seriennummer gespeichert wird, ohne zuvor alle eingetragenen Seriennummern zu löschen (Schritt 145). In einem weiteren Prozess P11 erfolg dann als nächstes eine Erfolgsmeldung (Schritt 147) und eine Abfrage dahingehend, ob alle Stelleinrichtungen angemeldet sind (Schritt 149). Ist dies nicht der Fall, so schreitet das Verfahren zu dem bereits zuvor beschrieben Prozess 12 voran, in dem die vorgegebene Zeitspanne wiederhergestellt wird, bevor das Verfahren zu Prozess P4 zurückkehrt. Wird in Schritt 149 hingegen festgestellt, dass alle Stelleinrichtungen angemeldet sind, so erhöht Prozess P11 in Schritt 151 die Sendeleistung auf ihren Ausgangswert, so dass die ursprüngliche, für den regulären Betrieb vorgesehene Sendeleistung wieder hergestellt wird. In Schritt 153 wird dann das Blinken der LED aufgehoben, und das Verfahren kehrt zum Leerlaufprozess P0 zurück.

Mit der Anmeldung der letzten Stelleinrichtung ist das Anmeldeverfahren beendet.

Bisher wurden mit Bezug auf Figur 3 die im Rahmen des Anmeldeverfahrens in der Steuereinheit 16 des Stativs 1 ablaufenden Prozesse beschrieben. Mit Bezug auf Figur 4 werden nunmehr die während des Anmeldeverfahrens im Fußschaltpult 2 ablaufenden Prozesse beschrieben. Zur Unterscheidung der im Fußschaltpult 2 ablaufenden Prozesse von den in der Steuereinheit 16 ablaufenden Prozesse werden die im Fußschaltpult 2 ablaufenden Prozesse jeweils als Prozess P' bezeichnet.

Zur Beginn des Anmeldeverfahrens befindet sich das Fußschaltpult 2 in einem Leerlaufprozess, der in Figur 4 als Prozess P'0 bezeichnet ist. In diesem Leerlaufprozess erfolgt in regelmäßigen Abständen eine Abfrage, ob von einem medizinischen Gerät, im vorliegenden Ausführungsbeispiel vom Stativ 1, ein Sammelaufruf zur Anmeldung am Stativ 1 gesendet worden ist (Schritt 201). Wird ein solcher Sammelaufruf nicht empfangen, so verbleibt das Fußschaltpult 2 im Leerlaufprozess P'0. Ist hingegen ein Sammelaufruf empfangen worden, so schreitet das Verfahren zu Schritt 203 voran, in dem abgefragt wird, ob eine Freigabetaste am Fußschaltpult 2 betätigt ist. Ist dies nicht der Fall, so verbleibt das Verfahren im Leerlaufprozess P'0. Ist die Freigabetaste hingegen betätigt, so schreitet das Verfahren zu Prozess P'1 fort.

Prozess P'1 führt zuerst eine Abfrage dahingehend durch, ob die Freigabetaste noch betätigt ist. Ist dies nicht der Fall, so kehrt das Verfahren in den Leerlaufprozess P'0 zurück. Falls die Freigabetaste noch betätigt ist, so erfolgt in Schritt 207 eine Abfrage dahingehend, ob fünf Sekunden verstrichen sind. Ist dies nicht der Fall, so kehrt das Verfahren in den Ausgangszustand des Prozesses P'1 zurück, so dass in Schritt 205 eine erneute Abfrage erfolgen kann, ob die Freigabetaste noch bestätigt ist. Auf diese Weise erfolgt eine Freigabe für die Durchführung der Anmeldeprozedur nur dann, wenn die Freigabetaste mindestens fünf Sekundenlang betätigt worden ist. Ist dies nicht der Fall, so wird in Schritt 205 festgestellt werden, dass die Taste nicht mehr betätigt ist, so dass das Verfahren in den Leerlaufprozesses P'0 zurückkehrt. Durch die Maßnahme, dass die Freigabetaste mindestens fünf Sekundenlang betätigt sein muss, kann vermieden werden, dass durch ein unbeabsichtigtes Betätigen der Freigabetaste, welches in der Regel nicht über einen Zeitraum von fünf Sekunden erfolgt, eine unbeabsichtigte Anmeldung des Fußschaltpultes 2 an einem medizinischen Gerät erfolgt.

Wenn die Freigabetaste länger als fünf Sekunden betätigt worden ist, so wird im Prozess P'2 die Sendeleistung des Fußschaltpultes 2 soweit verringert, dass das gesendete Signal nur in seinem Nahbereich empfangen werden kann (Schritt 209). Danach schreitet das Verfahren zu Prozess P'3 fort. Prozess P'3 führt die eigentliche Anmeldeprozedur aus, in der Daten mit der Steuereinheit 16 des Stativs 1 ausgetauscht werden. Im Rahmen dieses Datenaustausches werden die Seriennummern des Stativs bzw. seiner Steuereinheit 16 und dem Fußschaltpult 2 als Identifikationscodes ausgetauscht. Nachdem die Daten in Schritt 211 ausgetauscht worden sind, wird in Schritt 213 überprüft, ob Fehler aufgetreten sind. Ist dies der Fall, so schreitet Prozess P'3 zu einem Fehlerbehandlungszustand 215 fort, in dem überprüft werden kann welche Fehler aufgetreten sind und der eine Fehlerbehebung zulässt.

Sind in Schritt 213 hingegen keine Fehler beim Datenaustausch festgestellt worden, so schreitet das Verfahren zu Prozess P'4 fort, in dem die Seriennummer des Stativs bzw. seiner Steuereinheit 16 in einem nicht flüchtigen Speicher gespeichert wird. Die Vorteile des Speicherns im nicht flüchtigen Speicher sind bereits mit Bezug auf den Prozess P8, der in der Steuereinheit 16 des Stativs 1 während des Anmeldeverfahrens abläuft, beschrieben worden.

Nachdem in Schritt 217 die Seriennummer des Stativs bzw. seiner Steuereinheit 16 gespeichert worden ist, schreitet das Anmeldeverfahren zu Prozess P'5 fort, in dem die Sendeleistung des Fußschaltpultes 2 wieder auf seinen ursprünglichen, für den regulären Betrieb vorgesehenen Wert angehoben wird (Schritt 219). Danach wird in Schritt 221 eine Erfolgsmeldung ausgegeben, bevor das Verfahren wieder in den Leerlaufprozess P'0 zurückkehrt.

Für das Fußschaltpult 2 ist das Anmeldeverfahren damit abgeschlossen. Das Anmeldeverfahren wird auch in der Steuereinheit 16 abgeschlossen, wenn dort in Schritt 149 festgestellt worden ist, dass alle Stelleinrichtungen angemeldet sind. Sind hingegen noch nicht alle Stelleinrichtungen angemeldet, so wird das Anmeldeverfahren von der Steuereinheit 16 solange weitergeführt, bis alle Stelleinrichtungen angemeldet sind.

Nachdem das Anmeldeverfahren sowohl beim Fußschaltpult 2 als auch am Stativ 1 beendet worden ist, kann der reguläre Betrieb aufgenommen werden. Bei jeder Übertragung von Daten werden dann die gespeicherten identifikationscodes, also im vorliegenden Ausführungsbeispiel die gespeicherten Seriennummern, als Adresse mit übertragen, damit der Sender und der Empfänger der Daten identifiziert werden können.

Im erfindungsgemäßen Anmeldeverfahren bleibt eine Zuordnung solang erhalten, bis ein neues Anmeldeverfahren durchgeführt wird, wobei dann im Stativ 1 im Prozess P8 alle zuvor eingetragenen Seriennummern gelöscht werden. Mittels des Prozesses P1 können aber alle Seriennummern auch ohne eine eigentliche Anmeldeprozedur durchzuführen gelöscht werden, so dass alle Zuordnungen zu dem entsprechenden medizinischen Gerät aufgehoben werden, ohne dass neue Zuordnungen erstellt werden.

Dadurch, dass die Dauer des in den Prozessen P3 und P4 zyklisch gesendeten Sammelaufrufs zeitlich begrenzt ist, wird die Wahrscheinlichkeit, dass eine Stelleinrichtung versehentlich an einem medizinischen Gerät angemeldet wird, begrenzt. Zudem werden durch das Absenken der Sendeleistung auf eine niedrige Leistungsstufe nur solche Stelleinrichtungen angesprochen, die sich in unmittelbarer Nähe des zur Anmeldung aufrufenden medizinischen Geräts befinden. Der Blinkcode der LED gibt zudem den Nutzer Auskunft darüber, dass sich das entsprechende Gerät im Anmeldeverfahren befindet.

Zudem wird im Unterschied zum Stand der Technik das Anmeldeverfahren im Rahmen der Erfindung nicht von der Stelleinrichtung 2 initiiert, sondern von der Steuereinheit 16 des medizinischen Gerätes. Eine Anmeldung der Stelleinrichtung 2 kann daher nur an einem solchen medizinischen Gerät erfolgen, das ein Anmeldeverfahren initiiert hat. Andere medizinische Geräte stehen der Stelleinrichtung für das Durchführen eines Anmeldeverfahrens hingegen nicht zur Verfügung. Auch dadurch kann die Wahrscheinlichkeit einer Fehlanmeldung reduziert werden.

Weiterhin muss eine Zuordnung einer Stelleinrichtung zu einem medizinischen Gerät nicht wiederholt werden, bevor ein neuer Anmeldevorgang initiiert wird. Aufgrund der nicht flüchtigen Speicherung der Identifikationscodes bleibt die Zuordnung nämlich auch dann erhalten, wenn die Stromversorgung zum medizinischen Gerät und/oder zur Stelleinrichtung unterbrochen wird oder wenn die kabellose Kommunikationsverbindung unterbrochen wird. Die Zuordnung wird erst dann aufgehoben, wenn ein neuer Anmeldevorgang durchgeführt wird, oder die Aufhebung der Zuordnung durch Betätigen einer Taste gewollt herbeigeführt wird. Ein Nutzer muss deshalb nicht bei jedem Einschalten des medizinischen Gerätes, jedem Batteriewechsel, etc. oder immer wenn die Kommunikation kurzfristig unterbrochen war, einen neuen Anmeldevorgang starten.

Insgesamt ist durch das erfindungsgemäße Verfahren die Gefahr, dass bei Einsatz mehrerer Stelleinrichtungen in der näheren Umgebung eines medizinischen Gerätes diesem eine falsche Stelleinrichtung zugeordnet wird, gegenüber dem Stand der Technik reduziert.

## Patentansprüche

1. Verfahren zum Anmelden von kabellosen elektrischen Stelleinrichtungen (2) an einem medizinischen Gerät (1) über einen kabellosen Kommunikationskanal mit den Schritten: Initiieren des Anmeldevorgangs für eine kabellose elektrische Stelleinrichtung (2) an einem medizinischen Gerät (1) und Durchführen einer Anmeldeprozedur, in der die Stelleinrichtung (2) dem medizinischen Gerät (1) zugeordnet wird, wobei die Initiierung des Anmeldevorgangs vom medizinischen Gerät (1) aus erfolgt und die Sendeleistung des medizinischen Geräts (1) und der Stelleinrichtung (2) während des Anmeldevorgangs verringert ist, wobei das Initiieren des Anmeldevorgangs ein Absenken der Sendeleistung des Senders (17) des medizinischen Geräts (1) und ein anschließendes Aussenden eines Aufrufs zum Durchführen der Anmeldeprozedur mit abgesenkter Sendeleistung auslöst und bei Empfang des Aufrufs zum Durchführen der Anmeldeprozedur durch eine Stelleinrichtung (2) die Sendeleistung des Senders (19) der Stelleinrichtung (2) verringert wird, bevor diese eine Antwort auf den Aufruf sendet und die Anmeldeprozedur durchgeführt wird.

2. Verfahren nach Anspruch 1, **dadurch gekennzeichnet, dass** der Aufruf zum Durchführen der Anmeldeprozedur ein Sammelaufruf ist.

3. Verfahren nach Anspruch 1 oder Anspruch 2, **dadurch gekennzeichnet, dass** das Verringern der Sendeleistung und das Antworten auf den Aufruf nur erfolgt, wenn eine Anmeldefreigabe durch einen Nutzer der Stelleinrichtung (2) erfolgt.

4. Verfahren nach einem der vorangehenden Ansprüche, **dadurch gekennzeichnet, dass** eine bestehende Zuordnung von Stelleinrichtungen (2) im medizinischen Gerät (1) aufgehoben wird, wenn vom medizinischen Gerät (1) eine Antwort empfangen wird.

5. Verfahren nach einem der vorangehenden Ansprüche, **dadurch gekennzeichnet, dass** der Anmeldevorgang vom medizinischen Gerät (1) beendet wird, wenn innerhalb einer vorgegebenen Zeitspanne keine Stelleinrichtung (2) auf den Aufruf des medizinischen Geräts (1) antwortet.

6. Verfahren nach einem der vorangehenden Ansprüche, **dadurch gekennzeichnet, dass** während der Anmeldeprozedur Identifikationscodes ausgetauscht werden.

7. Verfahren nach Anspruch 6, **dadurch gekennzeichnet, dass** die jeweiligen Identifikationscodes im medizinischen Gerät (1) und/oder in der Stelleinrichtung (2) in einem nicht flüchtigen Speicher gespeichert werden.

8. Verfahren nach einem der vorangehenden Ansprüche, **dadurch gekennzeichnet, dass** im medizinischen Gerät (1) die Zuordnung bereits am medizinischen Gerät (1) angemeldeter Stelleinrichtungen (2) erhalten bleibt, wenn eine weitere Stelleinrichtung (2) am medizinischen Gerät (1) angemeldet wird.

9. Verfahren nach Anspruch 8, **dadurch gekennzeichnet, dass** im medizinischen Gerät (1) die Zuordnung aller am medizinischen Gerät (1) angemeldeten Stelleinrichtungen (2) auf einen Aufhebungsbefehl hin aufgehoben wird.

10. Verfahren nach einem der vorangehenden Ansprüche, **dadurch gekennzeichnet, dass** die Sendeleistung des medizinischen Geräts (1) und/oder der Stelleinrichtung (2) nach Abschluss der Anmeldeprozedur wieder erhöht wird.

## Claims

1. Method for pairing wireless electrical actuators (2) with a medical device (1) via a wireless communication channel having the steps: initiation of the pairing operation for a wireless electrical actuator (2) with a medical device (1) and performing of a pairing procedure, in which the actuator (2) is assigned to the medical device (1), wherein the initiation of the pairing operation takes place from the medical device (1) and the transmitting power of the medical device (1) and of the actuator (2) is reduced during the pairing operation, wherein the initiation of the pairing operation triggers a lowering of the transmitting power of the transmitter (17) of the medical device (1) and a subsequent emission of a request to perform the pairing procedure with a reduced transmitting power and on receipt of the request to perform the pairing procedure by an actuator (2) the transmitting power of the transmitter (19) of the actuator (2) is reduced, before this sends a response to the request and the pairing procedure is performed.

2. Method according to claim 1, **characterised in that** the request to perform the pairing procedure is a broadcast request.

3. Method according to claim 1 or claim 2, **characterised in that** the reduction in the transmitting power and the response to the request take place only when pairing is enabled by a user of the actuator (2).

4. Method according to one of the preceding claims, **characterised in that** an existing assignment of actuators (2) in the medical device (1) is cancelled when a response is received by the medical device (1).

5. Method according to one of the preceding claims, **characterised in that** the pairing operation is ended by the medical device (1) if no actuator (2) responds to the request from the medical device (1) within a predetermined period of time.

6. Method according to one of the preceding claims, **characterised in that** identification codes are exchanged during the pairing procedure.

7. Method according to claim 6, **characterised in that** the respective identification codes are stored in the medical device (1) and/or in the actuator (2) in a non-volatile memory.

8. Method according to one of the preceding claims, **characterised in that** the assignment of actuators (2) already paired with the medical device (1) is retained in the medical device (1) when a further actuator (2) is paired with the medical device (1).

9. Method according to claim 8, **characterised in that** the assignment of all the actuators (2) paired with the medical device (1) is cancelled in the medical device (1) in response to a cancel command.

10. Method according to one of the preceding claims, **characterised in that** the transmitting power of the medical device (1) and/or of the actuator (2) is increased again after conclusion of the pairing procedure.

## Revendications

1. Procédé de reconnaissance mutuelle d'actionneurs (2) électriques sans fil et d'un appareil médical (1) via un canal de communication sans fil, comprenant les étapes suivantes : initiation du processus de reconnaissance mutuelle d'un actionneur (2) électrique sans fil et d'un appareil médical (1) et exécution d'une procédure de reconnaissance mutuelle dans laquelle l'actionneur (2) est associé à l'appareil médical (1), l'initiation du processus de reconnaissance mutuelle s'opérant à partir de l'appareil médical (1) et la puissance d'émission de l'appareil médical (1) et de l'actionneur (2) étant réduite au cours du processus de reconnaissance mutuelle, l'initiation du processus de reconnaissance mutuelle déclenchant une diminution de la puissance d'émission de l'émetteur (17) de l'appareil médical (1), suivie de l'émission d'un appel à exécuter la procédure de reconnaissance mutuelle avec une puissance d'émission réduite et à réduire, à réception de l'appel à exécuter la procédure de reconnaissance mutuelle par un actionneur (2), la puissance d'émission de l'émetteur (19) de l'actionneur (2) avant que celui-ci n'émette une réponse à l'appel et que la procédure de reconnaissance mutuelle ne soit exécutée.

2. Procédé selon la revendication 1, **caractérisé en ce que** l'appel à exécuter la procédure de reconnaissance mutuelle est une diffusion.

3. Procédé selon la revendication 1 ou 2, **caractérisé en ce que** la diminution de la puissance d'émission et la réponse à l'appel ne se produisent que si une autorisation de reconnaissance mutuelle est donnée par un utilisateur de l'actionneur (2).

4. Procédé selon l'une des revendications précédentes, **caractérisé en ce qu'**une association existante d'actionneurs (2) à l'appareil médical (1) est annulée lorsqu'une réponse (1) est reçue par l'appareil médical (1).

5. Procédé selon l'une des revendications précédentes, **caractérisé en ce qu'**il est mis fin au processus de reconnaissance mutuelle de l'appareil médical (1) quand aucun actionneur (2) ne répond à l'appel de l'appareil médical (1) dans un laps de temps prédéterminé.

6. Procédé selon l'une des revendications précédentes, **caractérisé en ce que** des codes d'identification sont échangés au cours de la procédure de reconnaissance mutuelle.

7. Procédé selon la revendication 6, **caractérisé en ce que** les codes d'identification respectifs dans l'appareil médical (1) et/ou dans l'actionneur (2) sont stockés dans une mémoire non volatile.

8. Procédé selon l'une des revendications précédentes, **caractérisé en ce que**, dans l'appareil médical (1), l'association d'actionneurs (2) déjà connectés à l'appareil médical (1) reste maintenue lorsqu'un autre actionneur (2) est associé à l'appareil médical (1).

9. Procédé selon la revendication 8, **caractérisé en ce que**, dans l'appareil médical (1), l'association de tous les actionneurs (2) associés à l'appareil médical (1) est annulée par une commande d'annulation.

10. Procédé selon l'une des revendications précédentes, **caractérisé en ce que** la puissance d'émission de l'appareil médical (1) et/ou de l'actionneur (2) est de nouveau augmentée après la fin de la procédure de reconnaissance mutuelle.
